# EUROPEAN PATENT SPECIFICATION

(11) **EP 1 424 992 B1**
(45) Date of publication and mention of the grant of the patent: **19.10.2005**
(21) Application number: 02772155.4
(22) Date of filing: 15.08.2002
(51) Int. Cl.: A61K 7/48

(54) **THICKENER SYSTEM FOR COSMETIC COMPOSITIONS**
VERDICKUNGSSYSTEM FÜR KOSMETISCHE ZUSAMMENSETZUNGEN
SYSTEME EPAISSISSANT POUR COMPOSITIONS COSMETIQUES

(30) Priority: 12.09.2001 US 318660 P
(43) Date of publication of application: 09.06.2004
(73) Proprietor: UNILEVER PLC, London EC4P 4BQ (GB); UNILEVER N.V., 3013 AL Rotterdam (NL)
(72) Inventor: ZHANG, Joanna H., Unilever Home & Pers. Care USA, Trumbull, CT 06611 (US); SUARES, Alan J. Unilever Home & Personal Care USA, Trumbull, CT 06611 (US)
(74) Representative: Acham, Nicholas Clive
(86) International application number: PCT/EP2002/009118
(87) International publication number: WO 2003/022237

(56) References cited:
- EP-A- 0 987 014
- EP-A- 1 216 686
- WO-A-01/28552
- WO-A-01/91703
- WO-A-94/18935
- US-A- 4 540 510

## Description

The invention relates to improved thickening systems for cosmetic compositions, particularly those in lotion and cream form.

Aqueous cosmetic compositions often require thickeners to achieve an aesthetically pleasing viscosity. Fluids that flow with a watery consistency too rapidly run off the treated skin areas. For a cosmetic to be effective, it often must have substantivity. Thickeners provide this substantivity. Furthermore, low viscosity formulas which may be skin effective nevertheless through their wateriness signal ineffectiveness to the consumer. Products of watery consistency are also aesthetically displeasing to consumers with expectations of rich and creamy products.

U.S. Patent 5,422,112 (Williams) discloses a triple thickener system including xanthan gum, magnesium aluminum silicate and polyacrylamide. The compositions are said to be particularly effective for thickening alpha-hydroxy carboxylic acids and salts thereof, especially at low pH.

U.S. Patent 5,874,095 (Deckner et al.) reports an enhanced skin penetration system for improved topical delivery of drugs. Essential to the system is a nonionic polyacrylamide of high molecular weight described as effective at low pH.

U.S. Patent 5,952,395 (Lorant) and U.S. Patent 5,891,452 (Sebillote-Arnaud et al.) describe cosmetic compositions gelled into an emulsion with a cross-linked poly(2-acrylamido-2-methylpropanesulfonic acid).

Countless numbers of other thickening agents are known in the literature. Perhaps this plethora intimates that not all thickening agents are equally effective for any particular type of formulation.

Indeed, there are some formulations which are extremely difficult to thicken, and even if initially thickened may have storage stability problems. Low pH systems are particularly sensitive and difficult.

Accordingly, it is an advantage of the present invention to provide a thickener system and thickened cosmetic compositions of sufficiently aesthetically pleasing viscosity and skin feel.

It is another advantage of the present invention to provide thickening systems for cosmetic compositions that are effective at low pH.

It is still another advantage of the present invention to provide thickening systems for water and oil emulsion cosmetic compositions that also function as stabilizers preventing phase separation.

These and other advantages of the present invention will more readily become apparent from the description and examples which follow.

A cosmetic composition is provided which includes:
(i) from 0.001 to 5% by weight of a polysaccharide gum;
(ii) from 0.001 to 10% by weight of a taurate copolymer;
(iii) from 0.01 to 20% by weight of a C₁-C₂₅ alpha-hydroxycarboxylic acid; and
(iv) a cosmetically acceptable carrier, wherein the composition has a pH of less than 7.

Now it has been discovered that taurate copolymers in combination with polysaccharide gums are highly effective thickening agents for low pH cosmetic compositions. This system is particularly useful for building viscosity in relatively acidic compositions containing C₁-C₂₅ alpha-hydroxycarboxylic acids. Beyond building viscosity, the thickening system of this invention has the further advantage of stabilizing oil and water emulsions and providing a good skin feel.

Accordingly, a first element of compositions according to the present invention is that of a taurate copolymer. A particularly preferred copolymer is one wherein the taurate repeating monomer unit is acryloyl dimethyltaurate (in either free acid or salt form). Monomers forming the copolymer with taurate may include: styrene, acrylic acid, methacrylic acid, vinyl chloride, vinyl acetate, vinyl pyrrolidone, isoprene, vinyl alcohol, vinyl methylether, chloro-styrene, dialkylamino-styrene, maleic acid, acrylamide, methacrylamide and mixtures thereof. Where the term "acid" appears, the term means not only the free acid but also C₁-C₃₀ alkyl esters, anhydrides and salts thereof. Preferably but not exclusively the salts may be ammonium, alkanolammonium, alkali metal and alkaline earth metal salts.

Most preferred as the copolymer is Acryloyl Dimethyltaurate/Vinyl Pyrrolidone Copolymer, which is the INCI nomenclature, for a material supplied by Clariant Corporation under the trademark Aristoflex® AVC, having the following general formula: 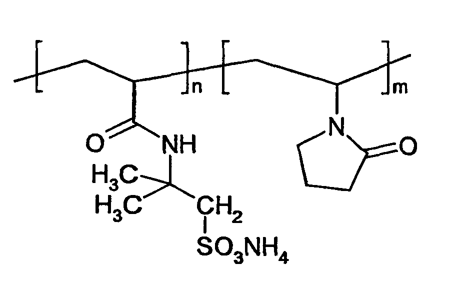 wherein n and m are integers which may independently vary from 1 to 10,000.

Average molecular weight of copolymers according to the invention may range from 1,000 to 3,000,000, preferably from 3,000 to 100,000, optimally from 10,000 to 80,000.

Amounts of the taurate copolymer may range from 0.001 to 10%, preferably from 0.01 to 8%, more preferably from 0.1 to 5%, optimally from 0.2 to 1% by weight of the composition.

A second element of compositions according to the present invention is that of a polysaccharide. Examples include natural or synthetic varieties of starches, gums and cellulosics. Representative of the starches are chemically modified starches such as aluminum starch octenylsuccinate. Suitable gums include xanthan, sclerotium, pectin, karaya, arabic, gelatin, agar, guar, carrageenan, alginate and combinations thereof. Suitable cellulosics include hydroxyethyl cellulose, hydroxypropyl cellulose, hydroxypropyl methylcellulose, methylcellulose, ethylcellulose and sodium carboxy methylcellulose. Most preferred is aluminum starch octenylsuccinate.

Amounts of the polysaccharide may range from 0.001 to 5%, preferably from 0.1 to 2%, optimally from 0.2 to 0.5% by weight.

Compositions according to the present invention contain an alpha-hydroxycarboxylic acid. The former may be a C₁-C₂₅ alpha-hydroxycarboxylic acid of formula I 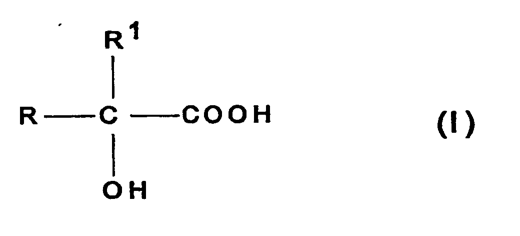 wherein R and R¹ are independently H, F, Cl, Br, alkyl, aralkyl or aryl groups being saturated or unsaturated, isomeric or nonisomeric, straight or branched chain, or in cyclic form having 5 or 6 ring members, and in addition, R and R¹ may carry OH, CHO, COOH and alkoxy groups having 1 to 9 carbon atoms, the α-hydroxyacid existing as a free acid or lactone form, or in salt form with an organic amine base or an inorganic alkali, and as stereoisomers, and D, L, and DL forms when R and R¹ are not identical.

Illustrative of this group of materials are 2-hydroxyethanoic acid (glycolic acid); 2-hydroxypropanoic acid (lactic acid); 2-methyl 2-hydroxypropanoic acid (methyllactic acid); 2-hydroxybutanoic acid; 2-hydroxypentanoic acid; 2-hydroxyhexanoic acid; 2-hydroxyheptanoic acid; 2-hydroxyoctanoic acid; 2-hydroxynonanoic acid; 2-hydroxydecanoic acid; 2-hydroxyundecanoic acid; 2-hydroxydodecanoic acid (α-hydroxylauric acid); 2-hydroxytetradecanoic acid (α-hydroxymyristic acid); 2-hydroxyhexadecanoic acid (α-hydroxypalmitic acid); 2-hydroxyoctadecanoic acid (α-hydroxystearic acid); 2-hydroxyeicosanoic acid (α-hydroxyarachidonic acid); 2-phenyl 2-hydroxyethanoic acid (mandelic acid); 2,2-diphenyl 2-hydroxyethanoic acid (benzilic acid); 3-phenyl 2-hydroxypropanoic acid (phenyllactic acid); 2-phenyl 2-methyl 2-hydroxyethanoic acid (atrolactic acid); 2-(4'-hydroxyphenyl) 2-hydroxyethanoic acid; 2-(4'-chlorophenyl 2-hydroxyethanoic acid; 2-(3'-hydroxy-4'-methoxyphenyl) 2-hydroxyethanoic acid; 2-(4'-hydroxy-3'-methoxyphenyl) 2-hydroxyethanoic acid; 3-(2-hydroxyphenyl) 2-hydroxypropanoic acid; 3-(4'-hydroxyphenyl) 2-hydroxypropanoic acid; and 2-(3',4'-dihydroxyphenyl) 2-hydroxyethanoic acid.

Most preferred of this group of materials are glycolic acid, lactic acid, gluconolactone and 2-hydroxyoctanoic acids. The term "acid" is here again intended to refer to not only the free acid form but also the esters, lactone and salt forms. The salts may be selected from alkalimetal, ammonium and C₁-C₂₀ alkyl or alkanolammonium counterions. The compositions of the present invention may also comprise a beta-hydroxy-carboxylic acid. The beta-hydroxycarboxylic acids are best illustrated by salicylic acid and its derivatives. Levels of the hydroxycarboxylic acids may range from 0.01 to 20%, preferably from 0.2 to 10%, optimally from 1 to 5% by weight.

Compositions of the present invention may either be aqueous or anhydrous. Preferably the compositions are aqueous, especially water and oil emulsions of the W/O or O/W variety. Water when present will be in amounts which may range from 5 to 90%, preferably from 35 to 70%, optimally between 40 and 60% by weight.

The pH of aqueous compositions of this invention will be less than 7. Advantageously, pH may range from 1 to 6, preferably from 2 to 5.5, optimally from 2.5 to 3.8.

Besides water, relatively volatile solvents may also be incorporated within compositions of the present invention.

Most preferred are monohydric C₁-C₃ alkanols. These include ethyl alcohol, methyl alcohol and isopropyl alcohol. The amount of monohydric alkanol may range from 5 to 50%, preferably from 15 to 40%, optimally between 25 to 35% by weight.

Emollient materials in the form of mineral oils, silicone oils and synthetic esters may be incorporated into compositions of the present invention. Amounts of the emollients may range anywhere from 0.1 to 30%, preferably between 0.5 and 20% by weight.

Silicone oils may be divided into the volatile and non-volatile variety. The term "volatile" as used herein refers to those materials which have a measurable vapor pressure at ambient temperature. Volatile silicone oils are preferably chosen from cyclic or linear polydimethylsiloxanes containing from 3 to 9, preferably from 4 to 5, silicon atoms.

Linear volatile silicone materials generally have viscosities less than 5 centistokes at 25°C while cyclic materials typically have viscosities of less than 10 centistokes.

Nonvolatile silicone oils useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from 5 to 100,000 centistokes at 25°C.

Among suitable ester emollients are:
(1) Alkenyl or alkyl esters of fatty acids having 10 to 20 carbon atoms. Examples thereof include isopropyl palmitate, isopropyl isostearate, isononyl isonanonoate, oleyl myristate, oleyl stearate, and oleyl oleate.
(2) Ether-esters such as fatty acid esters of ethoxylated fatty alcohols.
(3) Polyhydric alcohol esters. Ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters.
(4) Wax esters such as beeswax, spermaceti, myristyl myristate, stearyl stearate.
(5) Sterols esters, of which soya sterol and cholesterol fatty acid esters are examples thereof.

The most preferred esters are dicaprylyl ether and isopropyl isostearate.

Fatty acids having from 10 to 30 carbon atoms may also be included in the compositions of this invention. Illustrative of this category are pelargonic, lauric, myristic, palmitic, stearic, isostearic, hydroxystearic, oleic, linoleic, ricinoleic, arachidic, behenic and erucic acids.

Humectants of the polyhydric alcohol-type may also be included in the compositions of this invention. The humectant aids in increasing the effectiveness of the emollient, reduces scaling, stimulates removal of built-up scale and improves skin feel. Typical polyhydric alcohols include glycerol (also known as glycerin), polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2,6-hexanetriol, ethoxylated glycerol, propoxylated glycerol and mixtures thereof. For best results the humectant is preferably glycerin. The amount of humectant may range anywhere from 0.5 to 30%, preferably between 1 and 15% by weight of the composition.

Collectively the water, solvents, silicones, esters, fatty acids and/or humectants are viewed as cosmetically acceptable carriers for the compositions of the invention. Total amount of carrier will range from 1 to 99.9%, preferably from 80 to 99% by weight.

Cosmetic compositions of the present invention may be in any form. These forms may include lotions, creams, roll-on formulations, mousses, aerosol sprays and cloth- or pad-applied formulations.

Emulsifiers may also be present in cosmetic compositions of the present invention. Total concentration of the emulsifier will typically range from 0.1 to 40%, preferably from 1 to 20%, optimally from 1 to 5% by weight of the total composition. The emulsifier may be selected from the group consisting of anionic, nonionic, cationic and amphoteric actives. Particularly preferred nonionic surfactants are those with a C₁₀-C₂₀ fatty alcohol or acid hydrophobe condensed with from about 2 to about 100 moles of ethylene oxide or propylene oxide per mole of hydrophobe; C₂-C₁₀ alkyl phenols condensed with from 2 to 20 moles of alkylene oxide; mono- and di- fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g. methyl gluconamides) are also suitable nonionic emulsifiers.

Preferred anionic emulsifiers include soap, alkyl ether sulfate and sulfonates, alkyl sulfates and sulfonates, alkylbenzene sulfonates, alkyl and dialkyl sulfosuccinates, C₈-C₂₀ acyl isethionates, C₈-C₂₀ alkyl ether phosphates, alkylethercarboxylates and combinations thereof.

Sunscreen actives may also be included in compositions of the present invention. Particularly preferred are such materials as Avobenzene, available as Parsol® 1789, ethylhexyl p-methoxycinnamate, available as Parsol® MCX, and benzophenone-3, also known as Oxybenzone. Inorganic sunscreen actives may be employed such as microfine titanium dioxide, polyethylene and various other polymers. If incorporated, the amounts of the sunscreen agents will generally range from 0.1 to 30%, preferably from 2 to 20%, optimally from 4 to 10% by weight.

Preservatives can desirably be incorporated into the cosmetic compositions of this invention to protect against the growth of potentially harmful microorganisms. Suitable traditional preservatives for compositions of this invention are alkyl esters of parahydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are iodopropynyl butyl carbamate, phenoxyethanol, methyl paraben, propyl paraben, imidazolidinyl urea, sodium dehydroacetate and benzyl alcohol. The preservatives should be selected having regard for the use of the composition and possible incompatibilities between the preservatives and other ingredients in the emulsion. Preservatives are preferably employed in amounts ranging from 0.01% to 2% by weight of the composition.

For additional thickening, it is preferred to have magnesium aluminum silicate, commercially available as Veegum®, sold by the R.T. Vanderbilt Company. Amounts of this inorganic thickening agent may range from 0.01 to 10%, preferably from 0.5 to 1.2% by weight.

Minor adjunct ingredients may also be present in the cosmetic compositions. Among them may be the water-insoluble vitamins such as Vitamin A Palmitate, Vitamin E Acetate and DL-panthenol. Also useful are: retinol, ceramides and herbal extracts including green tea and chamomile.

Colorants, fragrances and abrasives may also be included in compositions of the present invention. Each of these substances may range from 0.05 to 5%, preferably between 0.1 and 3% by weight.

Except in the operating and comparative examples, or where otherwise explicitly indicated, all numbers in this description indicating amounts of material ought to be understood as modified by the word "about".

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

The following Examples will more fully illustrate the embodiments of this invention. All parts, percentages and proportions referred to herein and in the appended claims are by weight unless otherwise indicated.

### EXAMPLES 1-8

Typical formulations according to the present invention are described below.

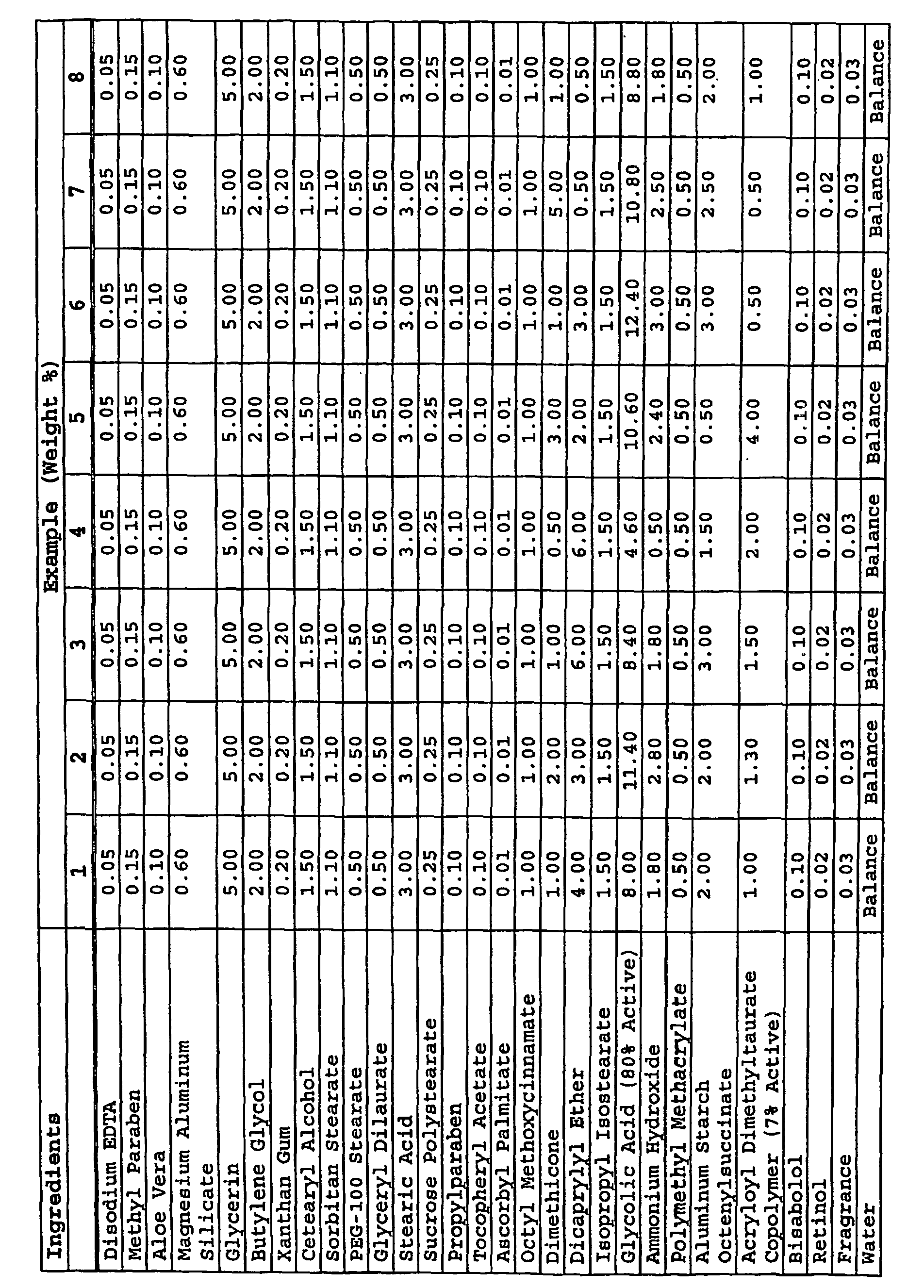

### EXAMPLE 9

A series of comparative experiments were conducted to measure the relative thickening effectiveness of various polysaccharides and synthetic polymers in low pH systems. Materials utilized in the comparative experiments are identified below.

| **Thickener** | **INCI Name** |
|---|---|
| Keltrol® | Xanthan gum |
| Natrosol ® 250 HHR | Hydroxyethyl cellulose |
| Solanace® | Potato Starch Modified |
| Sepigel® 305 | polyacrylamide/C13-14 Isoparaffin/Laureth-7 |
| Carbopol® 934 | Crosslinked Polyacrylate |
| Aristoflex® AVC | Acryloyl Taurate/Vinyl Pyrrolidone Copolymer |

All viscosity measurements were conducted using a Brookfield RVT instrument, Spindle No. 4 at 20 rpm and 23°C.

Table 1 reports the effect of three polysaccharide thickeners. Keltrol® is representative of natural gums. Natrosol® 250 HHR is representative of chemically modified cellulosic gums. Solanace® is representative of modified starches. Each thickener was formulated at 1% in water to form a gel. Second and third columns report results on gels further including 8% glycolic acid (AHA) and 8% glycolic acid/ammonium glycolate (AHA-Ammonium), respectively. In the latter system, the acid to salt molar ratio was 3.4:1 to simulate electrolyte effects. None of the polysaccharide thickeners by themselves achieve viscosities much beyond 5,000 cps, at low pH.

**TABLE 1**

| Polysaccharide Thickener | | | | | | |
|---|---|---|---|---|---|---|
| | **1% Gel** | | **1% Gel + 8% AHA** | | **1% Gel + 8% AHA-Ammonia** | |
| **Thickener** | **Viscosity (cps)** | **pH** | **Viscosity (cps)** | **pH** | **Viscosity (cps)** | **pH** |
| Keltrol® | 1,600 | 5.50 | 5,170 | 2.00 | 3,540 | 3.62 |
| Natrosol® | 2,580 | 6.80 | 2,290 | 1.90 | 2,130 | 3.63 |
| Solanace® | 327 | 8.20 | <100 | 1.90 | <100 | 3.62 |

Table 2 reports the effect on thickening of three synthetic polymers, including Aristoflex® AVC representative of taurate polymers of the present invention. Table 3 reports on gel compositions identical to those tested in Table 2 except the synthetic polymers have been increased to the 2% level.

**TABLE 2**

| Polymer Thickener | | | | | | |
|---|---|---|---|---|---|---|
| | **1% Gel** | | **1% Gel + 8% AHA** | | **1% Gel + 8% AHA-Ammonia** | |
| **Thickener** | **Viscosity (cps)** | **pH** | **Viscosity (cps)** | **pH** | **Viscosity (cps)** | **pH** |
| Sepigel ® 305 | 5,400 | 6.30 | <1,000 | 1.84 | <100 | 3.61 |
| Carbopol® 934 | 152 | 2.90 | <100 | 1.84 | <100 | 3.60 |
| Aristoflex® AVC | 48,000 | 4.82 | 1,380 | 1.96 | <100 | 3.64 |

**TABLE 3**

| Polymer Thickener | | | | | | |
|---|---|---|---|---|---|---|
| | **2% Gel** | | **2% Gel + 8% AHA** | | **2% Gel + 8% AHA-Ammonia** | |
| **Thickener** | **Viscosity (cps)** | **pH** | **Viscosity (cps)** | **pH** | **Viscosity (cps)** | **pH** |
| Sepigel ® 305 | 19,995 | 6.00 | <1000 | 2.00 | <100 | 3.6 |
| Carbopol® 934 | 1,735 | 2.80 | <100 | 1.90 | <100 | 3.5 |
| Aristoflex® AVC | 40,000 | 4.80 | 20,500 | 2.00 | 2,555 | 3.6 |

Table 4 lists the viscosity effect of combining 1% each of the polysaccharide thickeners with Sepigel® 305. Table 5 reports the combination of the polysaccharides each with Carbopol® 934. Finally, Table 6 reports results according to the present invention wherein the polysaccharides are matched with Aristoflex® AVC.

**TABLE 4**

| Sepigel® + Polysaccharide | | | | | | |
|---|---|---|---|---|---|---|
| | **+ 1% + 1%** | | **1% + 1% + 8% AHA** | | **1% + 1% + 8% AHA-Ammonia** | |
| **Thickener** | **Viscosity (cps)** | **pH** | **Viscosity (cps)** | **pH** | **Viscosity (cps)** | **pH** |
| Sepigel® + Keltrol® | 3,740 | 5.30 | 10,000 | 2.00 | 6,110 | 3.61 |
| Sepigel® + Natrosol® | 31,050 | 6.50 | 10,380 | 1.86 | 3,980 | 3.60 |
| Sepigel® + Solanace® | 13,150 | 8.14 | 195 | 1.89 | 250 | 3.61 |

**TABLE 5**

| Carbopol® + Polysaccharide | | | | | | |
|---|---|---|---|---|---|---|
| | 1**% + 1%** | | **1% + 1% + 8% AHA** | | **1% + 1% + 8% AHA-Ammonia** | |
| **Thickener** | **Viscosity (cps)** | **pH** | **Viscosity (cps)** | **pH** | **Viscosity (cps)** | **PH** |
| Carbopol® + Keltrol® | 5,420 | 3.43 | 7,350 | 1.97 | 5,950 | 3.58 |
| Carbopol® + Natrosol® | 350 | 2.98 | 240 | 1.85 | 4,050 | 3.57 |
| Carbopol® + Solanace | 20,700 | 3.28 | 210 | 1.88 | 200 | 3.58 |

**TABLE 6**

| Aristoflex® + Polysaccharide | | | | | | |
|---|---|---|---|---|---|---|
| | **1% + 1%** | | **1% + 1% + 8% AHA** | | **1% + 1% + 8% AHA-Ammonia** | |
| **Thickener** | **Viscosity (cps)** | **pH** | **Viscosity (cps)** | **pH** | **Viscosity (cps)** | **pH** |
| Aristoflex® + Xanthan Gum | 10,500 | 5.16 | 18,700 | 2.06 | 10,940 | 3.65 |
| Aristoflex® + Natrosol® 250 HHR | 61,000 | 5.79 | 24,400 | 1.94 | 10,380 | 3.60 |
| Aristoflex® + Solanace® | 36,200 | 7.17 | 3,490 | 1.95 | 2,610 | 3.60 |

Based on the results of the combinations reported in Table 4-6, it is evident that the taurate polymer (Aristoflex® AVC) under all low pH conditions, especially in the presence of electrolytes, provides higher viscosity gels with polysaccharides than do other common synthetic polymeric thickeners.

## Claims

1. A cosmetic composition comprising:
(i) from 0.001 to 5% by weight of a polysaccharide gum;
(ii) from 0.001 to 10% by weight of a taurate copolymer;
(iii) from 0.01 to 20 % by weight of C₁-C₂₅ alpha-hydroxy-carbonyl acid; and
(iv) a cosmetically acceptable carrier, wherein the composition has a pH of less than 7.

2. A composition according to claim 1 wherein the polysaccharide is selected from xanthan, selerorium, pectin, karaya, arabic, agar, guar, carrageenan, alginate and combinations thereof.

3. A composition according to claim 1 or claim 2 wherein the taurate copolymer is at least partially formed from acryloyl dimetyltaurate as a monomer unit.

4. A composition according to claim 3 wherein the acryloyl dimethyltaurate is copolymerized with a monomer selected from styrene, acrylic acid, methacrylic acid, vinyl chloride, vinyl acetate, vinyl pyrrolidone, isoprene, vinyl alcohol, vinyl methylether, chloro-styrene, maleic acid, acrylamide, methacrylamide and mixtures thereof.

5. A composition according to claim 4 wherein the taurate copolymer is acryloyl dimethyltaurate/vinyl pyrrolidone copolymer.

6. A composition according to any of the preceding claims wherein the polysaccharide is xanthan gum and the taurate copolymer is acryloyl dimethyltaurate/vinyl pyrrolidone copolymers.

7. A composition according to any of the preceding claims wherein the pH ranges from 1 to 6.

8. A composition according to claim 7 wherein the pH ranges from 2.5 to 3.8.

9. A composition according to any of the preceding claims wherein the polysaccharide ranges in amount from 0.1 to 2% and the taurate copolymer ranges in amount from 0.1 to 5%.

10. A composition according to any of preceding claims wherein the alpha-hydroxycarboxylic acid is selected from glycolic acid, lactic acid and 2-hydroxyoctanoic acid.

## Patentansprüche

1. Kosmetische Zusammensetzung, umfassend
(i) 0,001 bis 5 Gew.-% eines Polysaccharidgummistoffs;
(ii) 0,001 bis 10 Gew.-% eines Tauratcopolymers;
(iii) 0,01 bis 20 Gew.% einer C₁-C₂₅-α-Hydroxycarbonsäure und
(iv) einen kosmetisch verträglichen Träger,
wobei die Zusammensetzung einen pH von weniger als 7 hat.

2. Zusammensetzung nach Anspruch 1, wobei das Polysaccharid aus Xanthan, Sklerotium, Pektin, Karaya, Gummi arabicum, Agar, Guar, Carragheen, Alginat und Kombinationen davon ausgewählt wird.

3. Zusammensetzung nach Anspruch 1 oder Anspruch 2, wobei das Tauratcopolymer zumindest teilweise aus Acryloyldimethyltaurat als Monomereinheit hergestellt wird.

4. Zusammensetzung nach Anspruch 3, wobei das Acryloyldimethyltaurat mit einem aus Styrol, Acrylsäure, Methacrylsäure, Vinylchlorid, Vinylacetat, Vinylpyrrolidon, Isopren, Vinylalkohol, Vinylmethylether, Chlorstyrol, Maleinsäure, Acrylamid, Methacrylamid und Gemischen davon ausgewählten Monomer copolymerisiert wird.

5. Zusammensetzung nach Anspruch 4, wobei das Tauratcopolymer Acryloyldimethyltaurat/Vinylpyrrolidon-Copolymer ist.

6. Zusammensetzung nach einem der vorstehenden Ansprüche, wobei das Polysaccharid Xanthangummi und das Tauratcopolymer Acryloyldimethyltaurat/Vinylpyrrolidon-Copolymere ist.

7. Zusammensetzung nach einem der vorstehenden Ansprüche, wobei der pH im Bereich von 1 bis 6 liegt.

8. Zusammensetzung nach Anspruch 7, wobei der pH im Bereich von 2,5 bis 3,8 liegt.

9. Zusammensetzung nach einem der vorstehenden Ansprüche, wobei die Menge des Polysaccharids im Bereich von 0,1 bis 2 % und die Menge des Tauratcopolymers im Bereich von 0,1 bis 5 % liegt.

10. Zusammensetzung nach einem der vorstehenden Ansprüche, wobei die α-Hydroxycarbonsäure aus Glycolsäure, Milchsäure und 2-Hydroxyoctansäure ausgewählt wird.

## Revendications

1. Composition cosmétique comprenant :
(i) de 0,001 à 5 % en poids d'une gomme de polysaccharide ;
(ii) de 0,001 à 10 % en poids d'un copolymère de taurate ;
(iii) de 0,01 à 20 % en poids d'un acide C₁-C_{QS} alpha-hydroxy-carboxylique ; et
(iv) un support acceptable de façon cosmétique,
dans laquelle la composition a un pH de moins de 7.

2. Composition selon la revendication 1, dans laquelle le polysaccharide est sélectionné parmi le xanthane, le sclérote, la pectine, la gomme de karaya, la gomme arabique, l'agar, le guar, la carragénine, l'alginate et les combinaisons de ceux-ci.

3. Composition selon la revendication 1 ou la revendication 2, dans laquelle le copolymère de taurate est au moins partiellement formé à partir d'acryloyl diméthyltaurate en tant que motif monomère.

4. Composition selon la revendication 3, dans laquelle l'acryloyl diméthyltaurate est copolymérisé avec un monomère sélectionné parmi le styrène, l'acide acrylique, l'acide méthacrylique, le chlorure de vinyle, l'acétate de vinyle, la vinylpyrrolidone, l'isoprène, l'alcool vinylique, l'éther méthylvinylique, le chlorostyrène, l'acide maléique, l'acrylamide, le méthacrylamide et les mélanges de ceux-ci.

5. Composition selon la revendication 4, dans laquelle le copolymère de taurate est un copolymère d'acryloyl diméthyltaurate/vinylpyrrolidone.

6. Composition selon l'une quelconque des revendications précédentes, dans laquelle le polysaccharide est de la gomme de xanthane et le copolymère de taurate se compose de copolymères d'acryloyl diméthyltaurate/vinylpyrrolidone.

7. Composition selon l'une quelconque des revendications précédentes, dans laquelle le pH varie de 1 à 6.

8. Composition selon la revendication 7, dans laquelle le pH varie de 2,5 à 3,8.

9. Composition selon l'une quelconque des revendications précédentes, dans laquelle le polysaccharide varie en quantité de 0,1 à 2 % et le copolymère de taurate varie en quantité de 0,1 à 5 %.

10. Composition selon l'une quelconque des revendications précédentes, dans laquelle l'acide alpha-hydroxycarboxylique est sélectionné parmi l'acide glycolique, l'acide lactique et l'acide 2-hydroxyoctanoïque.
